(19) Europäisches Patentamt

European Patent Office

Office européen des brevets

(11) EP 0 680 339 B1

(12) EUROPEAN PATENT SPECIFICATION

(45) Date of publication and mention
of the grant of the patent:
**10.04.2002 Bulletin 2002/15**

(51) Int Cl.7: **A61K 47/14**, A61K 47/20,
A61K 47/40, A01N 25/02,
A61K 9/00

(21) Application number: **94904532.2**

(22) Date of filing: **18.01.1994**

(86) International application number:
**PCT/AU94/00023**

(87) International publication number:
**WO 94/16732 (04.08.1994 Gazette 1994/18)**

(54) **ECTOPARASITICIDAL FORMULATION**

EKTOPARASITIZIDE FORMULIERUNG

FORMULATION ECTOPARASITICIDE

(84) Designated Contracting States:
**BE DE DK ES FR GB GR IE IT NL PT SE**

(30) Priority: **20.01.1993 AU PL686293**

(43) Date of publication of application:
**08.11.1995 Bulletin 1995/45**

(73) Proprietors:
- **SOLTEC RESEARCH PTY. LTD.**
  **Rowville Victoria 3178 (AU)**
- **Novartis Animal Health Inc.**
  **4002 Basel (CH)**

(72) Inventors:
- **HALLS, Neil Graham**
  **Rowville, VIC 3178 (AU)**
- **KLOSE, John Barry**
  **Rowville, VIC 3178 (AU)**

(74) Representative: **Brown, David Leslie et al**
**Page Hargrave**
**Southgate**
**Whitefriars**
**Lewins Mead**
**Bristol BS1 2NT (GB)**

(56) References cited:
**EP-A- 0 273 862       AU-A- 6 111 390**
**AU-A- 7 401 887       AU-A- 9 185 082**
**AU-A- 9 185 182**

- **CHEMICAL ABSTRACTS, vol. 117, no. 25, 21
  December 1992 Columbus, Ohio, US; abstract
  no. 247162, & AU-B-627 847 (ANCARE
  DISTRIBUTORS LTD.,NZ) 3 September 1992**
- **CHEMICAL ABSTRACTS, vol. 107, no. ,
  Columbus, Ohio, US, page 288, column , abstract
  no. 193060, : "Liquid stabilized preparation
  containing as an active substance
  S-[n-(2-Chlorophenyl)butyramidomethyl]O,O-di
  met- hyl dithiophosphate, for use against
  ectoparasites of animals, 1987"**

## Description

<u>Technical Field</u>

This invention relates to pour-on formulations active against ectoparasites of sheep, as well as to their uses against ectoparasite infestation of sheep.

<u>Background Art</u>

Traditionally sheep have been treated against infestation by ectoparasites such as lice or blow fly by dipping the sheep in an ectoparasiticidal formulation so that the animal is totally immersed. The difficulty with this method is that high volumes of the ectoparasiticidal formulation are necessary, that it is difficult to encourage the sheep to enter the dip and that it is both time consuming and physically demanding. A further disadvantage is that large volumes of the "dip" have then to be disposed of in an environmentally correct fashion.

Hungarian Patent No. 41238 discloses a liquid stabilised emulsion, dilutable to provide a dipping composition for use against Psoroptes ectoparasites of sheep, containing a mixture of S-[N-(2-chlorophenyl)butyramidomethyl] O, O-dimethyl dithiophosphate (47.0%), dibutylphthalate (36.5%), isopropyl myristate (10.0%) and polyoxyethyl alkyl aryl ether-alkylsulfonate (6.5%). The pre-dilution emulsion contains 85% of the said mixture. The diluted emulsion contains 0.2% of the dithiophosphate active.

One alternative proposed to dipping animals is to apply an ectoparasiticidal formulation as a "pour-on", the formulation being applied locally, usually along the animal's backline. with the object that, by force of gravity, it spreads around the animal giving protection or treatment to all parts of the animal. One such formulation is disclosed in Australian Patent 563723 to Wellcome Australia Limited. This formulation comprises an anti-parasitic or insecticidal agent suspended or dispersed in an aqueous carrier.

The difficulty with some prior art formulations is that the carrier dries out too quickly and does not allow the active agent to spread across the entirety of the animal's body. Thus untreated areas remain on the animal allowing the infestation to quickly return to the treated parts of the body.

A number of Australian Patents including 77109/81, 61113/90, 599383 and 31919/77 also disclose pour-on formulations but do not specifically exemplify pour-on formulations for treating sheep.

It must be appreciated that the fleece of a sheep whether in a shorn or unshorn form presents a unique set of problems in the treatment of ectoparasites; problems which are quite distinct from those of cattle hide. In particular, in unshorn sheep, the size and bulk of the fleece prevents the formulation being able to reach all parts of the animals body which might be infested, specifically in the brisket and neck region and in the lower parts of the body where infestation is usually high due to the warm, moist environment and its distance from the conventional backline location of application. The size and bulk of a fleece are particularly a problem if organo phosphorus compounds are used as the active agent since these compounds do not readily translocate. For this reason organophosphorus compounds are not commonly used as pour-on ectoparasiticides for sheep.

Synthetic pyrethroids are becoming less effective as pour-on ectoparasiticides since the vermin are developing resistance to the common synthetic pyrethroids used.

The consequence of the growing resistance to pour-on formulations against ectoparasiticides based on synthetic pyrethroids is that they are increasingly ineffectual in eliminating infestation of sheep by ectoparasites. Because of the likely reinfestation of the animal subsequent to present treatments their economic benefit is limited. Desirably, such pour-on formulations should be 100% effective against the ectoparasite so that reinfestation is not such a rapid inevitability. The quality of the flock of sheep and thus the economic'benefit therefrom can be greatly improved if a 100% kill rate can be achieved.

It is thus an object of this invention to provide a pour-on formulation for treatment of ectoparasites in sheep which achieves a desired kilt rate, and preferably a 100% kill rate, by migration around the entirety of the animal's body.

It is a further objects of this invention to provide a use against ectoparasite infestation of sheep, which maximises the probability of migration of the active agent about the entire body surface of the animal.

<u>Summary of the invention</u>

This invention is predicated upon the discovery that the reason many ectoparasiticidal formulations do not reach all parts of the animal's body is that they dissolve the lanolin of the sheep's fleece. Thus, on application, they dissolve the lanolin and become "locked" in the fleece preventing their seepage about the animal's body. It has been found that if a solvent-carrier is selected in which lanolin is not dispeisible, the translocation of the active agent across the body of either a shorn or unshorn animal is greatly improved.

The invention relates to the use of an ectoparasiticidal active agent and a non-lanolin dissolving phthalate-comprising solvent-carrier therefor in the preparation of a pour-on ectoparasiticidal formulation for the pour-on treatment of ectoparasitically infested sheep, wherein the said active agent is present in said pour-on ectoparasiticidal formulation in an amount of 1 - 15 % w/w.

The invention further relates to the use of an ectoparasiticidal active agent and a physiologically acceptable non-lanolin dissolving phthalate-comprising solvent-carrier therefor in the preparation of a pour-on ectoparasiticidal formulation, characterised in that said active agent is present in an amount of 1-15 % w/w, for the treatment of ectoparasitically infested sheep by a method comprising pour-on administration of the formulation to a sheep in a volume greater that 1.0 ml per kilo bodyweight of the sheep such that an ectoparasiticidal kill rate as required by the national regulations of the country in which the formulation is to be used is achieved.

A pour-on formulation effective against ectoparasites of sheep according to another embodiment of the invention comprises an effective amount of at least one active agent in a non-lanolin dissolving solvent-carrier therefor, characterised in that the non-lanolin dissolving solvent-carrier; (a) has a viscosity low enough to enable translocation of the at least one active agent around the body of the sheep to be treated, and (b) comprises a phthalate; and further characterised in that said active agent is present in an amount of 1-15 % w/w.

A composition according to the invention may comprise an organophosphorus compound in a non-lanolin dissolving solvent-carrier therefor, for use as a pour-on ectoparasiticidal formulation for the treatment of ectoparasitically infested sheep, characterised in that the non-lanolin dissolving solvent-carrier: (a) has a viscosity low enough to enable translocation of the at least one active agent around the body of the sheep to be treated, and (b) comprises a phthalate; and further characterised in that said active agent is present in an amount of 1 - 15 % w/w.

Solvent-carriers which are most suitable for-use in the formulations of the invention are those characterized as not being able to dissolve in lanolin at 30°C, and as having a viscosity low enough to enable translocation of the active agent around the body of a sheep to be treated. Desirably the carrier-solvent is also non-toxic. This latter characteristic is important in the treatment of sheep which are to be slaughtered for human consumption.

Throughout this specification, the term "non-lanolin dissolving" is used with respect to solvent-carriers to mean those in which lanolin will not disperse at 30°C.

Several families of solvent-carriers may additionally be used in the formulation of the invention including vegetable-based esters, co-solvents soluble in both water and oils and low viscosity kerosene-like solvents. In particular, the more preferred solvent-carriers are those which are vegetable-based esters, phthalates or derivatives thereof.

Examples of suitable phthalates include dibutyl phthalate, di-2-ethylhexyl phthalate, di-iso-octyl phthalate, dimethyl phthalate, diethyl phthalate, di-isobutyl-phthalate, di-isodecyl phthalate or n-octyl n-decyl phthalate. Other suitable solvent-carriers include glyceryl tricaprylate caprate, peanut oil, sunflower oil, castor oil and PPG-15 stearyl ether.

Active agents which might be incorporated into the formulation of the invention include synthetic pyrethroids, organo phosphorus compounds and insect growth regulators such as Vetrazin ™ (cyromazine). Because of the tendency of synthetic pyrethroids to dissolve in the oils released from the sheeps body after shearing, and because of the increasing resistance to synthetic pyrethroids of ectoparasites to be treated, the more preferred active agents are organo phosphorus compounds, exemplified by diazinon and propetamphos.

Preferably, the formulations of the invention are formulated as emulsifiable concentrates. Purely aqueous formulations, it has been found, are less effective.

The formulations of the invention may additionally include conventional excipients such as dyes, for example, Macrolex Yellow, Waxoline Yellow, surfactants such as, for example, Alkanate CS, Teric™, Kenmat™, emulsifiers, plasticizers, antioxidants, disinfectants and other conventional formulating agents.

In formulations according to the invention, there may be present in the concentrated formulation 20-85% w/w of non-lanolin dissolving solvent-carrier, and 1.0-15.0% w/w of active agent.

In a preferred embodiment of the invention, the concentrate formulation used is as follows:

|  | %w/w | mass (g) |
| --- | --- | --- |
| Diazinon 90S Tech (active agent) | 9.878 | 10.342 |
| Teric 12A4 (surfactant) | 1.958 | 2.050 |
| Corflex 400 (di-butyl phthalate) | 68.510 | 71.730 |
| Alkanate CS (emulsifier) | 9.452 | 9.896 |
| Teric 200 (surfactant) | 9.452 | 9.896 |
| Waxoline Yellow 2GP-FW (dye) | 0.750 | 0.785 |
| TOTAL | 100.000 | 104.699 |

Diluted 1 volume concentrate plus 6 volumes water as a working solution.

The preferred formulation is as follows.

|  | %w/w | mass (g) |
|---|---|---|
| Diazinon 905 Tech (active agent) | 1.468 | 1.478 |
| Teric 12A4 (surfactant) | 0.291 | 0.292 |
| Corflex 400 (di-butyl phthalate) | 10.179 | 10.247 |
| Alkanate CS (emulsifier) | 1.404 | 1.414 |
| Teric 200 (surfactant) | 1.404 | 1.414 |
| Waxoline Yellow 2GP-FW (dye) | 0.111 | 0.112 |
| Water | 85.143 | 85.743 |
| TOTAL | 100.000 | 100.700 |

Differences in national regulatory environments mean that different levels of residual sheep ectoparasite infestation are considered acceptable in different countries of the world. Thus, for example, in Australia, in order for a sheep to be acceptably treated, a 100% kill rate must be achieved, i.e. no ectoparasite must remain alive on the treated animal. However, in the United Kingdom, levels lower than 100% are considered acceptable treatment of infestation. "Desired kill rate" when used throughout this specification is thus defined as that of the national regulations of the country concerned.

Thus in a second aspect of the invention, there is provided a method of treatment of sheep being ectoparasitically infested comprising administering to a sheep pour-on ectoparasiticidal formulation, such as, for example, that described hereinabove, in a volume such that the desired kill rate is attained.

The volume to be applied will vary according to the body surface area to weight ratio of the animal to be treated. For example, in Europe where animals are small in body surface area but nevertheless weighty, the preferred volume to be applied is one that is greater than 1.0 ml per kilo bodyweight of the sheep to be treated. In Australia, however, where sheep are larger in body surface area but no weightier than European sheep, the desired volume is one that is greater than 2.0 ml per kilo body weight of the sheep to be treated.

This aspect of the invention is predicated on the discovery that reinfestation of sheep currently occurs basically as a result of untreated ectoparasites remaining in the brisket and remote regions of the animal. By increasing the volume of application (but not necessarily the concentration of active) it has been found that there is a stronger likelihood that the desired kill rate will be attained because the remote regions of the sheep are also treated.

It will be appreciated that the upper limit of volume of emulsion applied will vary depending on the state of the fleece of the sheep. A heavily fleeced sheep will require a larger quantity of emulsion than a shorn sheep.

It will also be appreciated that excess volumes applied will only result in substantial run-off and hence wastage, and thus should be avoided.

Wherein the desired kill rate is defined by national regulation laws, in a preferred embodiment of the invention there is provided a method of treatment of sheep being ectoparasitically infested comprising administering to a sheep a pour-on ectoparasiticidal formulation such as, for example, that described hereinabove, wherein the active agent is present in an amount such that when delivered in the desired formulation volume, the desired kill rate is attained. The concentration will vary with active and the resistance of the ectoparasite to be treated.

For example, where the active agent is Diazinon, an organo phosphorus compound, the desired rate of application of the ectoparasiticidal formulation is about 40 mg/kg active agent delivered in a volume of about 3 ml per kilo body weight of the infested sheep. It will be appreciated that the necessary concentration of active will vary depending on its chemical nature. In the case of Propetamphos, the desired concentration is one which is greater than 15 mg/kg.

The longer an animal remains infested with ectoparasites, the more likely its fleece will be damaged due to the tendency of the animal to rub against objects to relieve irritation caused by infestation. It is thus desirable to eradicate infestation in as rapid a period as possible.

Thus in a third aspect of the invention, there is provided a method of treatment of sheep being ectoparasitically infested comprising administering to a sheep a pour-on formulation, such as, for example, that described hereinabove in a volume such that the desired kill rate, as defined hereinabove, is substantially achieved within 14 days after treatment. Preferably the volume applied is greater than 2.0 ml per kilo body weight of the sheep to be treated.

The formulations according to the invention can be applied to long-woolled (fully fleeced) or shorn sheep although it will be appreciated that since the formulation acts upon the skin of the sheep, to deliver the formulation to a fully fleeced sheep will require a considerable excess to allow for uptake of the formulation by the fleece itself.

Further embodiments of the invention will now be described by way of example.

Calculations are based on the assumption that sheep are used having an average weight of approximately 50 kg and "success" is designated as a kill rate of 100% given that all tests were carried out under Australian and New Zealand conditions.

Testing for ectoparasite infestation was carried out by counting the number of parasites present in 20 partings taken on each side of the sheep (40 partings in total).

**EXAMPLE 1 -** POUR-ON LOUSICIDE EFFICIENCY AGAINST DAMALINIA OVIS

<u>EXAMPLE 1A:</u> An emulsion was formulated according to the following:

| Ingredient | % w/w | mass (g) |
|---|---|---|
| Diazinon (90.0%) | 2.325 | 23.25 |
| Teric 12A4 | 0.50 | 5.0 |
| Macrolex Yellow 3G | 0.10 | 1.0 |
| Corflex 440 (di-isobutyl phthalate) | 16.975 | 169.75 |
| Alkanate CS | 2.5 | 25.0 |
| Teric 200 | 2.5 | 25.0 |
| BHA | 0.1 | 1.0 |
| DI water | 74.86 | 748.6 |
| Nipastat | 0.14 | 1.4 |
| | 100.0 | 1000.0 |

The pour-on emulsion (Diazinon 2.1% w/w) was applied in a volume of 1.67 ml/kg body weight as a band commencing high on the neck of the sheep and finishing at the butt of the tail. Further bands were applied to either side of the initial stripe and partially overlapping the first until the appropriate volume was delivered. The treatment was applied immediately after shearing as follows:

| Ear Tag No | Weight (kg) | Dose Vol (mls) | Dose (mg/kg) |
|---|---|---|---|
| 232 | 27 | 45 | 35 |
| 177 | 25 | 40 | 33.6 |
| 212 | 33 | 55 | 35 |
| 187 | 33 | 55 | 35 |
| 203 | 30 | 50 | 35 |
| 208 | 25 | Untreated | Controls |
| 181 | 27 | Untreated | Controls |
| 241 | 32 | Untreated | Controls |
| 246 | 21 | Untreated | Controls |
| 219 | 30 | Untreated | Controls |

Treatments were administered using the Protector - dial a dose, drench/pour-on gun.

Once treated, each group was penned separately in an electrified paddock where they were kept for the duration of the trial.

Lice counts were performed pre-shearing, 7, 14, 21, 28, 35 and 42 days after treatment, using the 20 partings per side technique.

Results were calculated from the mean number of lice in each group. Percentage reduction of the lice count in the lice group was used as a correction factor.

The formula used was as follows

$$\% \text{ reduction} = 100 \times (1 - T_2/C_2 \times C_1/T_1)$$

Where

$T_1$ = pre treatment lice count of the treated group
$T_2$ = post-treatment lice count of the treated group
$C_1$ = pre-treatment lice count of the control group
$C_2$ = post-treatment lice count of the control group

| Group | Pre Shear | 7 DAT | 14 DAT | 21 DAT | 28 DAT | 35 DAT | 42 DAT |
|---|---|---|---|---|---|---|---|
| Treated sheep | 362.8 | 31.2 | 3.2 | 0.2 | 0.2 | 0.4 | 0 |
| Control | 353.4 | 306.2 | 311 | 244 | 196 | 200.8 | 166.4 |
| % Reduction | - | 90 | 99 | 99.9 | 99.9 | 99.8 | 100 |

Eradication of the sheep body louse <u>D. ovis</u> was achieved at a kill rate of 100% after 42 days of treatment using the formulation of this example.

**EXAMPLE 1B:** To demonstrate the activity of active ingredients other than Diazinon, a formulation according to the following was prepared:

| Ingredient | % w/w | mass (g) |
|---|---|---|
| Teric 12A4 | 1.958 | 78.32 |
| Waxoline Yellow 2GP-FW | 0.375 | 15.00 |
| Corflex 400 | 73.824 | 2952.96 |
| Alkanate CS | 9.452 | 378.08 |
| Teric 200 | 9.452 | 378.08 |
| Propetamphos (90-91% w/w) | 4.939 | 197.56 |
| | 100.000 | 4000.00 |

The pour-on emulsion (diluted 1:6) was applied in a volume of 3 ml/kg body weight in a manner similar to that described in Example 1A in a concentration of 20 mg/kg.
Lice counts were performed pre-shearing 7, 14, 21, 28, 35 and 42 days after treatment using the 20 partings per side technique.
Using the calculation methods described in Example 1A the following results were achieved:

| Group | Pre Shear | 7 DAT | 14 DAT | 21 DAT | 28 DAT | 35 DAT | 42 DAT |
|---|---|---|---|---|---|---|---|
| Control | 444.2 | 365.2 | 324.8 | 227.4 | 193.8 | 178.4 | 130.6 |
| Treated sheep | 435.8 | 0.8 | 0 | 0 | 0 | 0 | 0 |
| % Reduction | - | 99.8 | 100 | 100 | 100 | 100 | 100 |

Eradication of the sheep body louse <u>D. ovis</u> was achieved at a kill rate of 100% after only 14 days of treatment using the formulation of this example.

**COMPARISON EXAMPLE 1 -** EXCLUSION OF PHTHALATE EXTENDER

As a point of comparison with the formulations described in Example 1, a formulation excluding the phthalate extender Corflex 440 (di-isobutyl phthalate) and in the absence of any other substitute extender, was prepared as follows:

| Ingredient | % w/w | mass (g) |
|---|---|---|
| Diazinon (91%) | 1.48 | 14.8 |
| Teric 12A4 | 0.29 | 2.9 |
| Macroiex Yellow 3G | 0.11 | 1.1 |
| Alkanate CS | 1.40 | 14.0 |

(continued)

| Ingredient | % w/w | mass (g) |
|---|---|---|
| Teric 200 | 1.40 | 14.0 |
| Water | 95.27 | 952.7 |
| Orthophenylphenol | 0.05 | 0.5 |
| | 100.00 | 1000.0 |

The formulation was applied immediately after shearing in a manner as described in Example 1 and in a volume of 150 ml/50 kg sheep.

Percentage reduction was calculated as in previous examples.

| Group Lice Counts | | | | | | | |
|---|---|---|---|---|---|---|---|
| Ear Tag No | Pre Shearing | 7 DAT | 14 DAT | 21 DAT | 28 DAT | 35 DAT | 42 DAT |
| 231 | 742 | 126 | DIED | | | | |
| 43 | 365 | 286 | 110 | 39 | 20 | 21 | 3 |
| 41 | 347 | 253 | 135 | 51 | 26 | 17 | 7 |
| 52 | 298 | 125 | 41 | 5 | 3 | 3 | 7 |
| 97 | 260 | 205 | 104 | 90 | 70 | 104 | 50 |
| Average | 402.4 | 199 | 97.5 | 47.7 | 29.8 | 36.3 | 16.8 |
| Kill rate | | 41.0% | 73% | 87.0% | 91.2% | 88% | 94% |
| CONTROLS | | | | | | | |
| 31 | 730 | 618 | 605 | 570 | 607 | 445 | 483 |
| 24 | 390 | 309 | 304 | 275 | 300 | 135 | 72 |
| 143 | 466 | 477 | 429 | 364 | 329 | 465 | 396 |
| 13 | 268 | 102 | 215 | 273 | 247 | 274 | 233 |
| 156 | 249 | 236 | 285 | 301 | 284 | 195 | 245 |
| Average | 420.6 | 348.4 | 367.6 | 396.6 | 353.4 | 302.8 | 284.6 |

It is clear that a satisfactory sustained kill rate was not achieved in the absence of a phthalate extender.

**COMPARISON EXAMPLE 2 -** SUBSTITUTION OF PHTHALATE EXTENDER BY ALTERNATIVE EXTENDER IN WHICH LANOLIN IS SOLUBLE

As a further point of comparison with the formulation described in Example 1 and to show the efficacy of an extender not soluble in lanolin, the following formulation was prepared:

| Ingredient | % w/w | mass (g) |
|---|---|---|
| Diazinon (90.0%) | 12.0 | 84.0 |
| Teric 12A4 | 2.1 | 14.7 |
| Macrolex Yellow 3G | 0.8 | 5.6 |
| Solvesso 150 (extender) | 65.1 | 455.7 |
| Alkanate CS | 10.0 | 70.0 |
| Teric 200 | 10.0 | 70.0 |
| | 100.0 | 700.0 |

The formulation was emulsified in a concentrate:water ratio of 1:3 such that the active was present in a concentration of 2.7% w/w.

(Diazinon pour on concentrate 10.8% w/w)

The emulsion was applied in a volume of 1.67 mL/kg body weight at the same time and in the same manner as described in Example 1, according to the following:

| Ear Tag No | Weight (kg) | Dose Vol (mL) | Dose (mg/kg) |
|------------|-------------|---------------|--------------|
| Y51 | 36 | 60 | 45.0 |
| Y59 | 48 | 80 | 45.0 |
| Y57 | 52 | 85 | 44.1 |
| Y52 | 35 | 60 | 46.3 |
| Y55 | 42 | 70 | 45.0 |
| Y53 | 40 | Untreated Controls | |
| Y54 | 39 | "        " | |
| Y60 | 46 | "        " | |
| Y58 | 37 | "        " | |
| Y56 | 41 | "        " | |

Treatments were administered using the Protector dial a dose drench/pour-on gun.

Once treated, each group was penned separately in an electrified paddock where they were kept for the duration of the trial.

Lice counts were performed pre-shearing 7, 14, 21, 28, 35 and 41 days after treatment using the 20 partings per side technique.

The formula used was as follows

$$\% \text{ reduction} = 100\,(1 - T_2/C_2 \times C_1/T_1)$$

Where

$T_1$ = pre treatment lice count of the treated group
$T_2$ = post-treatment lice count of the treated group
$C_1$ = pre-treatment lice count of the control group
$C_2$ = post-treatment lice count of the control group

| Group | Pre Shear | 7 DAT | 14 DAT | 21 DAT | 28 DAT | 35 DAT | 41 DAT |
|-------|-----------|-------|--------|--------|--------|--------|--------|
| Treated sheep | 286.0 | 154.8 | 50.8 | 21.4 | 21.2 | 7.8 | 8.0 |
| Control | 281.4 | 250.4 | 245.0 | 227.0 | 189.2 | 196.8 | 157.4 |
| % Reduction | - | 51.8 | 79.6 | 90.7 | 89.0 | 96.1 | 95.0 |

In the absence of a phthalate extender, using a conventional solvent - extender, Solvesso 150, as a replacement therefor, eradicatic. of D. ovis to a degree sufficient for Australian Standards was not achieved.

EXAMPLE 2 - INCREASED VOLUME OF APPLICATION

To test whether or not increased volume of application achieved a faster kill rate, an aqueous emulsion of Diazinon was prepared according to the following:

| Ingredient | % w/w | mass (g) |
|------------|-------|----------|
| Diazinon (910 g/kg) | 1.67 | 25.05 |
| Teric 12A4 | 0.29 | 4.35 |
| Macrolex Yellow 3G | 0.11 | 1.65 |
| Corflex 440 | 9.0 | 135.0 |
| Alkanate CS | 1.4 | 21.0 |
| Teric 200 | 1.4 | 21.0 |
| Water | 86.13 | 1291.95 |

(continued)

| Ingredient | % w/w | mass (g) |
|---|---|---|
|  | 100.00 | 1500.00 |

This formulation was applied in a volume of 3mL/kg body weight of animal, the Diazinon being present in a concentration of 1.5% w/w, the dose rate being approximately 45 mg/kg.

All sheep were treated in the same manner as described in Example 1 and immediately post-shearing.

| Ear Tag No | Treatment | Weight (kg) | DoseVol (mL) | Dose (mg/kg) |
|---|---|---|---|---|
| 90 | Test formulation | 45 | 135 | 45.0 |
| 244 | " | 50 | 150 | 45.0 |
| 281 | " | 49 | 150 | 45.9 |
| 148 | " | 47 | 140 | 44.7 |
| 1 | " | 47 | 140 | 44.7 |
| 61 | Control | 46 | - | - |
| 46 | " | 46 | - | - |
| 84 | " | 41 | - | - |
| 88 | " | 43 | - | - |
| 12 | " | 41 | - | - |

Treatments were applied using the NZ Protector dial-a-dose drench/pour-on gun.

Once treated, each group was penned separately in electrified paddocks where they remained for the duration of the trial.

Lice counts were performed pre-shearing 7, 14, 21, 29, 35, 42, 49 and 57 dat using the 20 partings per side technique.

Percentage reduction in lice counts was calculated using the change in lice counts for the control group as a correction factor.

The formula used to calculate % reduction was the same as that used in Example 1, with the following result:

| Mean Lice Counts - Days after Treatment (DAT) | | | | | | | | | |
|---|---|---|---|---|---|---|---|---|---|
|  | Pre-Shear | 7 DAT | 14 DAT | 21 DAT | 29 DAT | 35 DAT | 42 DAT | 49DAT | 57DAT |
| Treated sheep | 193.2 | 0.2 | 0 | 0 | 0 | 0 | 0.2 | 0 | 0 |
| Control | 185.0 | 100.8 | 72.0 | 50.4 | 58.8 | 56.6 | 52.6 | 48.4 | 43.0 |
| % Red'n | - | 99.8 | 100.0 | 100.0 | 100.0 | 100.0 | 99.6 | 100.0 | 100.0 |

Eradication of the sheep body louse D. ovis was achieved after 14 days showing that increased volume of application gives more expedient results.

**EXAMPLE 3 -** VARYING CONCENTRATION OF ACTIVE

In order to determine the minimum effective concentration of active agent possible upon application of 150 mL/50 kg sheep, the following formulations were prepared:

## Base formulation

| Ingredient | % w/w | mass (g) |
|---|---|---|
| Diazinon (91%) } Water | 87.8 | 5268.0 |
| Macrolex Yellow 3G | 0.11 | 6.6 |
| Corflex 440 | 9.00 | 540.0 |
| Alkanate CS | 1.40 | 84.0 |
| Teric 200 | 1.40 | 84.0 |
| Teric 12A4 | 0.29 | 17.4 |
| | 100.0 | 6000.0 |

| Example 3A | (40 mg/kg) | (1.33% Diazinon w/w) |
|---|---|---|
| base formulation | 12.2 | 122.0 |
| Diazinon (91%) | 1.47 | 14.7 |
| water | 86.33 | 863.3 |
| | 100.0 | 1000.0 |

| Example 3B | (35 mg/kg) | (1.16% Diazinon w/w) |
|---|---|---|
| base formulation | 12.2 | 122.0 |
| Diazinon (91%) | 1.28 | 12.8 |
| water | 86.52 | 865.2 |
| | 100.0 | 1000.0 |

| Example 3C | (30 mg/kg) | (1.0% Diazinon w/w) |
|---|---|---|
| base formulation | 12.2 | 122.0 |
| Diazinon (91%) | 1.10 | 11.0 |
| water | 86.70 | 867.0 |
| | 100.0 | 1000.0 |

| Example 3D | (25 mg/kg) | (0.83% Diazinon w/w) |
|---|---|---|
| base formulation | 12.2 | 122.0 |
| Diazinon (91%) | 0.92 | 9.2 |
| water | 86.88 | 868.8 |
| | 100.0 | 1000.0 |

| Example 3E | (20 mg/kg) | (0.66% Diazinon w/w) |
|---|---|---|
| base formulation | 12.2 | 122.0 |
| Diazinon (91%) | 0.73 | 7.3 |

(continued)

| Example 3E | (20 mg/kg) | (0.66% Diazinon w/w) |
|---|---|---|
| water | 87.07 | 870.7 |
| | 100.0 | 1000.0 |

For each of Examples 3A-E, the treatments were applied immediately after shearing along the backline in 4-6 strips (depending on the volume to be applied. Details are as follows:

| Ear Tag No | Treatment | Weight (kg) | Dose Vol (mL) | Dose (mg/kg) |
|---|---|---|---|---|
| 160 | Example 3A | 40 | 120 | 40 |
| 171 | " | 49 | 150 | 40 |
| 196 | " | 44 | 130 | 40 |
| 124 | " | 51 | 155 | 40 |
| 139 | " | 43 | 130 | 40 |
| | | | | |
| 170 | Example 3B | 47 | 140 | 35 |
| 197 | " | 45 | 135 | 35 |
| 271 | " | 43 | 130 | 35 |
| 66 | " | 43 | 130 | 35 |
| 135 | " | 42 | 125 | 35 |
| | | | | |
| 98 | Example 3C | 43 | 130 | 30 |
| 119 | " | 43 | 130 | 30 |
| 228 | " | 48 | 145 | 30 |
| 69 | " | 47 | 140 | 30 |
| 63 | " | 47 | 140 | 30 |
| | | | | |
| 155 | Example 3D | 43 | 130 | 25 |
| 237 | " | 52 | 155 | 25 |
| 136 | " | 44 | 130 | 25 |
| 283 | " | 42 | 125 | 25 |
| 193 | " | 44 | 130 | 25 |
| | | | | |
| 191 | Example 3E | 45 | 135 | 20 |
| 198 | " | 42 | 125 | 20 |
| 187 | " | 42 | 125 | 20 |
| 105 | " | 48 | 145 | 20 |
| 175 | " | 41 | 125 | 20 |
| | | | | |
| 82 | Control | 44 | - | - |
| 149 | " | 49 | - | - |
| 161 | " | 52 | - | - |
| 166 | " | 47 | - | - |
| 85 | " | 42 | - | - |

Treatments were applied using the NZ Protector dial-a-dose drench/pour-on gun.

Once treated, each group was penned separately in electrified paddocks where they were kept for the duration of the trial.

Lice counts were performed pre-shearing 7, 14, 21, 28, 35 and 42 days after treatment using the 20 partings per side technique.

Percentage reduction in lice counts was calculated using the change in lice counts for the control group as a correction factor, using the formula given in example 1.

| Mean Lice Counts - Days After Treatment (DAT) | | | | | | | |
|---|---|---|---|---|---|---|---|
| | Pre-Shear Lice Count | 7 DAT | 14 DAT | 20 DAT | 28 DAT | 35 DAT | 42 DAT |
| **Example 3A** | | | | | | | |
| Treated Sheep | 163.8 | 42.4 | 19.6 | 6.4 | 3.4 | 2.6 | 0 |
| Control | 141.2 | 66.8 | 112.8 | 92.6 | 73.8 | 81.8 | 58.8 |
| % Red'n | | 45.3 | 85.1 | 94.1 | 96.0 | 97.3 | 100.0 |
| **Example 3B** | | | | | | | |
| Treated Sheep | 161.2 | 1.2 | 0.4 | 0 | 0 | 0 | 0 |
| Control | 141.2 | 66.8 | 112.8 | 92.6 | 73.8 | 81.8 | 58.8 |
| % Red'n | | 98.4 | 99.7 | 100.0 | 100.0 | 100.0 | 100.0 |
| **Example 3C** | | | | | | | |
| Treated Sheep | 150.8 | 14.6 | 2.4 | 0.8 | 0.8 | 0.6 | 0 |
| Control | 141.2 | 66.8 | 112.8 | 92.6 | 73.8 | 81.8 | 58.8 |
| % Red'n | | 79.6 | 98.0 | 99.2 | 98.9 | 99.3 | 100.0 |
| **Example 3D** | | | | | | | |
| Treated Sheep | 143.8 | 212.8 | 7.6 | 0.2 | 3.2 | 3.8 | 1.8 |
| Control | 141.2 | 66.8 | 112.8 | 92.6 | 73.8 | 81.8 | 58.8 |
| % Red'n | | 66.6 | 93.4 | 99.8 | 95.8 | 95.5 | 97.1 |
| **Example 3E** | | | | | | | |
| Treated Sheep | 140.2 | 5.2 | 1.2 | 0.2 | 0 | 0 | 0.2 |
| Control | 141.2 | 66.8 | 112.8 | 92.6 | 73.8 | 81.8 | 58.8 |
| % Red'n | | 92.2 | 98.9 | 99.8 | 100.0 | 100.0 | 99.7 |

Eradication of sheep body louse was achieved at 42 days using formulations 3A, 3B and 3C. Thus it is clear that these formulations are effective in achieving 100% kill rate, although higher concentrations are likely to give more expedient results, i.e. 100% kill in a lesser period of time.

In the Example 3A group, one animal was responsible for the delay in achieving 100% efficacy. The counts at 7, 14, 21, 28, 35 and 42 days post-treatment were 185, 94, 31, 17, 13 and 0 respectively. This sheep was extremely heavily infested and was difficult to shear; i.e. tufts of wool were present at treatment.

Similar tests using concentrations of Diazinon firstly at 15 mg/kg (0.5% w/w) and secondly at 10 mg/kg (0.33% w/w) failed to eradicate the sheep body louse D. ovis.

**EXAMPLE 4** - COMPARISON OF PHTHALATE EXTENDERS

To show that phthalate extenders other than di-isobutyl phthalate are effective for the purposes of the invention, the following formulations were prepared:

**EXAMPLE 4A**

| Ingredient | % w/w | mass (g) |
|---|---|---|
| Diazinon (91%) | 1.47 | 16.17 |
| Teric 12A4 | 0.29 | 3.19 |
| Macrolex Yellow 3G | 0.11 | 1.21 |

(continued)

| Ingredient | % w/w | mass (g) |
|---|---|---|
| Corflex 400 (di-butyl phthalate) | 9.00 | 99.0 |
| Alkanate CS | 1.40 | 15.4 |
| Teric 200 | 1.40 | 14.0 |
| Deionised Water | 86.28 | 949.08 |
| Orthophenylphenol | 0.05 | 0.55 |
| | 100.00 | 1100.0 |
| (1.33% Diazinon w/w; dose = 3.0 mL/kg ≡ 40 mg/kg) | | |

## EXAMPLE 4B

| Ingredient | % w/w | mass (g) |
|---|---|---|
| Diazinon (91%) | 1.47 | 16.17 |
| Teric 12A4 | 0.29 | 3.19 |
| Macrolex Yellow 3G | 0.11 | 1.21 |
| DIBP (di-isobutyl phthalate) | 9.00 | 99.00 |
| Alkanate CS | 1.40 | 15.40 |
| Teric 200 | 1.40 | 15.40 |
| Deionised Water | 86.28 | 949.08 |
| Orthophenylphenol | 0.05 | 0.55 |
| | 100.0 | 1100.0 |
| (1.33% Diazinon w/w; dose = 3.0 mL/kg ≡ 40 mg/kg) | | |

All sheep were treated immediately after shearing according to the manner described in Example 1. No control group was used.

| Ear Tag No | Treatment | Weight (kg) | Dose Vol (mL) | Dose (mg/kg) |
|---|---|---|---|---|
| 263 | Example 4A | 27 | 80 | 39.4 |
| 201 | " | 39 | 115 | 39.2 |
| 279 | " | 33 | 100 | 40.3 |
| 95 | " | 37 | 110 | 39.5 |
| 61 | " | 41 | 125 | 40.6 |
| 227 | Example 4B | 34 | 100 | 39.1 |
| 242 | " | 30 | 90 | 39.9 |
| 205 | " | 34 | 100 | 39.1 |
| 125 | " | 34 | 100 | 39.1 |
| 46 | " | 40 | 120 | 39.9 |

Treatments were applied using the NZ Protector dial-a-dose drench pour-on gun.

Once treated, each group was penned separately under cover for 9 days, after which they were penned outside in electrified paddocks where they were kept for the duration of the trial.

Lice counts were performed pre-shearing, 7, 14, 21, 28, 35 and 43 DAT using the 20 partings per side technique.

Percentage lice reductions were calculated by dividing the average pre-shearing lice count into the average weekly lice count for that group.

| Group Mean Lice Counts | | | | | | | |
|---|---|---|---|---|---|---|---|
| | Pre-Shear | 7 DAT | 14 DAT | 21 DAT | 28 DAT | 35 DAT | 43 DAT |
| **Example 4A** | | | | | | | |
| Treated Sheep | 353.6 | 27.0 | 6.8 | 3.0 | 1.60 | 0.2 | 0 |
| % Red'n | | 92.0 | 98.0 | 99.0 | 99.5 | 99.9 | 100 |
| | | | | | | | |
| **Example 4B** | | | | | | | |
| Treated Sheep | 348.6 | 14.2 | 4.6 | 0 | 0 | 0 | 0.2 |
| % Red'n | | 95.9 | 98.6 | 100 | 100 | 100 | 99.9 |

It is evident from these results that the formulations of Examples 4A and 4B are equivalent showing that dibutyl phthalate is an acceptable substitute for di-isobutyl phthalate.

**EXAMPLE 5** - FIELD TESTS

To determine large scale efficacy of formulations according to the invention a formulation was prepared as follows:

| Ingredient | % w/w | mass (g) |
|---|---|---|
| Diazinon 90S (Technical) (90.5%) | 9.878 | 29.634 |
| Teric 12A4 | 1.958 | 5.874 |
| Macrolex Yellow 3G | 0.743 | 2.229 |
| Corflex 400 | | |
| (dibutyl phthalate) | 68.517 | 205.551 |
| Kemmat SC15 | 9.452 | 28.356 |
| Teric 200 | 9.452 | 28.356 |
| | 100.000 | 300.000 |
| (diluted to 1 vol. conc.: 6 vol water to give Diazinon 1.33% w/w) | | |

Formulation was applied to sheep using a Magrath electric hand wand using 10 cm perforated brass "T" piece screw on nozzle.

A total of 901 sheep were treated immediately off shears as follows:

Prior to shearing, 25 2½ - 5½ year old merino ewes were randomly selected, ear tagged and counted for lice. A post-shearing lice count was then conducted.

The sheep were then weighed and dosed immediately after shearing according to the weight of the heaviest sheep in each mob i.e.,

| BODY WEIGHT - KG | DOSE VOLUME - mL |
|---|---|
| 0-30 | 90 |
| 31-40 | 120 |
| 41-50 | 150 |
| 51-60 | 180 |
| 61-70 | 210 |
| 71-75 | 225 |

Sheep in excess of 75 kg body weight to be dosed at 3.0 mL per kg live weight.

The appropriate dose was applied to the backline of each sheep in the mob in a single unbroken band approximately 120 mm wide, extending from behind the ears through to the butt of the tail.

TABLE 1

| MOB | NO. TREATED | HEAVIEST B/W RECORDED (Shorn) (kg) | DOSE VOL (mL) |
|---|---|---|---|
| Weaners | 159 | 47 | 150 |
| 1½ y/o ewes | 113 | 55 | 180 |
| 2½ - 5½ y/o ewes | 601 | 57 | 180 |
| 2½ y/o wethers | 28 | 65 | 210 |

Lice counts were performed using the 20 partings per side technique as follows:

| Pre-shearing | 25 eartag sheep | |
|---|---|---|
| Post-shearing | 25 eartag sheep | |
| 34 DAT | 25 eartag sheep | + 25 others at random* |
| 85 DAT | 25 eartag sheep | + 25 others at random* |
| 134 DAT | 25 eartag sheep | + 25 others at random* |
| 190 DAT | 25 eartag sheep | + 25 others at random* |

* Any sheep showing signs of rubbing are to be included in this group of 25.

The average number of lice found per sheep in each group was as follows:

TABLE 2

| | Pre-Shear | Post-Shear | 34 DAT | 85 DAT | 134 DAT | 190 DAT |
|---|---|---|---|---|---|---|
| 25 Eartag sheep | 146.6 | 71.7 | 0 | 0 | 0 | 0 |
| 25 Others | | | 0 | 0 | 0 | 0 |

Evidently eradication of D. ovis was achieved using the formulation of this example. No lice were found at subsequent inspections.

## EXAMPLE 6 - DEGREE OF TRANSLOCATION

To determine the degree and speed of translocation of the formulation of the invention over the body surface of a sheep after treatment, the following formulation was prepared and administered:

| | %w/w | mass (g) |
|---|---|---|
| Diazinon 90S Tech | 9.878 | 3.75 |
| Teric 12A4 | 1.958 | 0.74 |
| Corflex 400 | 68.510 | 26.04 |
| Kenmat | 9.452 | 3.59 |
| Teric 200 | 9.452 | 3.59 |
| Waxoline Yellow 2GP-FW | 0.750 | 0.29 |
| | 100.000 | 38.00 |
| (diluted 1:6 by volume to give Diazinon 1.33% w/w) | | |

A 3 year old merino was shorn, weighed and treated immediately with the diazinon spray-on. It had not received any insecticide treatment for 12 months. The sheep weighed 37 kg and was treated with 110mL of the product. A wide band was applied from high on the neck, behind the ears, to the butt of the tail at a dosage rate of 3mL per kg bodyweight.
The animal was hand restrained during treatment and placed under cover in a pen with mesh floor and sides for the duration of the trial.
During and after treatment, the sheep had no contact with other treated animals and was not exposed to any rain.
On advice from the State Chemistry Laboratory, Department of Agriculture, Victoria, approximately 2cm square scrapings of wool and skin were taken as follows:

| DAYS 0& DAYS 1.3.7 | 15 minutes after treatment<br>2 samples, left and right in the middle of the back and in the band of product. |
|---|---|
| DAYS 1. 3. 7 | Mid-line - half way from the backline to the bellyline, at the line of the shoulder, mid-flank and rump ie., 6 samples at each time period. This was repeated on the other side of the body ie., 12 samples in total. |
| DAYS 1, 3, 7 | Bellyline - at the line of the shoulder, mid-flank and rump on both sides ie., 12 samples at each time period. |
| DAYS 1, 3, 7 | A scraping was taken from the neck/brisket area, in the mid-line - see above diagram.<br><br>Care was taken in positioning the scrapings eg., left bellyline shoulder sample was exactly vertically below the mid-line shoulder sample at day 1, so that samples at days 3 and 7 were minimally affected by variations in diazinon spread caused by bare patches above the lower samples at days 3 and 7.<br><br>A new scalpel blade was used for each scraping site. |

Tables 3, 4 and 5 show the levels of diazinon detected at each test point throughout the trial.

## Table 3 - DIAZINON (PPM) WHOLE WOOL

| DAYS POST TREATMENT | BACKLINE | | MID-LINE LEFT | | |
|---|---|---|---|---|---|
| | LEFT | RIGHT | SHOULDER | FLANK | RUMP |
| 0 | 21,000 | 16,000 | | | |
| 1 | 19,000 | 19,000 | 1400 | 84 | 940 |
| 3 | 15,000 | 16,000 | 1100 | 220 | 170 |
| 7 | 21,000 | 17,000 | 730 | 190 | 590 |

| DAYS POST TREATMENT | MID-LINE RIGHT | | | BELLY-LINE LEFT | | |
|---|---|---|---|---|---|---|
| | SHOULDER | FLANK | RUMP | SHOULDER | FLANK | RUMP |
| 0 | | | | | | |
| 1 | 290 | 230 | 470 | 180 | 150 | 890 |
| 3 | 320 | 170 | 480 | 140 | 360 | 54 |
| 7 | 210 | 110 | 390 | 290 | 150 | 420 |

| DAYS POST TREATMENT | BELLY-LINE RIGHT | | | NECK |
|---|---|---|---|---|
| | SHOULDER | FLANK | RUMP | |
| 0 | | | | |
| 1 | 430 | 87 | 53 | 230 |
| 3 | 210 | 54 | 73 | 220 |
| 7 | 530 | 52 | 65 | 170 |

Table 4 -

| DIAZINON (PPM) - WHOLE WOLL | | | | |
|---|---|---|---|---|
| DAYS POST TREATMENT | MIDLINE | | BELLY-LINE | |
| | MEAN | RANGE | MEAN | RANGE |
| 1 | 569 | 84-1400 | 298 | 53-890 |
| 3 | 410 | 170-1100 | 148.5 | 54-360 |
| 7 | 370 | 110-730 | 251.2 | 52-530 |

Table 5 -

| DIAZINON (PPM) - WHOLE WOLL (MEAN VALUES) | | | | |
|---|---|---|---|---|
| DAYS POST TREATMENT | MIDLINE | | BELLY-LINE | |
| | LEFT | RIGHT | LEFT | RIGHT |
| 1 | 808 | 330 | 407 | 190 |
| 3 | 496 | 323 | 185 | 112 |
| 7 | 503 | 237 | 287 | 216 |

The values (PPM diazinon) are reported on a whole wool basis.

The laboratory determined the percentage wool grease on 3 samples - 16.11%, 21.5% and 17.6% w/w.

The approximate concentration in wool grease can be calculated on the basis of the average wool grease content of the wool is 18.4% w/w ie., multiply the individual results by a factor of 5.4.

Due to difficulties in handling the trial animal, the band applied did deviate somewhat to the left side of the mid-line. However, this would be seen in a commercial situation on some sheep and highlights the worth of a wide band of product being applied. The levels detected on the right belly-line at the flank and rump levels for days 1, 3 and 7 demonstrate the effect of the deviation of the band to the left. However, there was still ample coverage of this right side eg., Day 1, "belly-line right rump" 53 PPM diazinon in whole wool, or 286 PPM in the wool grease and presumably the skin fats and oils, where the lice live and breed.

Particular note is made of the fact that levels detected in the neck/brisket region at all stages was high illustrating the ability of the formulation to translocate across the entire animal.

A wide variation in the range of diazinon levels is noted; translocation would be affected by length of wool after shearing, conditions of the skin, wool follicle density, wool and skin grease levels and the level applied at the backline. At day 7, this range had tightened considerably.

**EXAMPLE 7 -** LONG-WOOLLED SHEEP

The following trials conducted in New Zealand demonstrate that the formulations according to the invention may also be useful against ectoparasite infestation of long-woolled sheep.

The following formulation was prepared and administered to Romney/Border Leicester ewes having 10-12 cm of wool growth.

| | %w/w | mass (kg) |
|---|---|---|
| Diazinon 90S Technical | 9.878 | 29.634 |
| Teric 12A4 | 1.958 | 5.874 |
| Macro-lex Yellow 3G | 0.743 | 2.229 |
| Corflex 400 | 68.517 | 205.551 |
| Kenmat SC15 | 9.452 | 28.356 |
| Teric 200 | 9.452 | 28.356 |
| TOTAL | 100.000 | 300.700 |
| (diluted 1:6 by volume to give Diazinon 1.33% w/w : rate = 3ml/ kg) | | |

The ewes were sprayed along the backline using a Protector gun and narrow nozzle. The treatment extended from the base of the neck to the lump in a 15 cm wide band.

The following results were achieved when a procedure of counting the lice in a 10 cm parting of wool at 10 sites around the body (being the brisket, neck, lower and upper shoulders and withers on each side of the body) was adopted.

| Ear Tag No. | Weight (kg) | Dose Vol (mls) | Louse Scores D.A.T. | | |
|---|---|---|---|---|---|
| | | | 0 | 21 | 41 |
| 370 | 67 | 201 | 9 | 3 | 0 |
| 371 | 80 | 240 | 11 | 0 | 0 |
| 372 | 78 | 234 | 11 | 1 | 0 |
| 373 | 53 | 165 | 17 | DEAD | |
| 374 | 73 | 219 | 13 | 0 | 0 |
| 375 | 73 | 219 | 7 | 0 | 0 |
| 497 | 67 | 201 | 62 | 1 | 0 |
| 498 | 78 | 234 | 35 | 0 | 0 |
| 499 | 85 | 255 | 21 | 0 | 0 |
| 500 | 80 | 240 | 45 | 1 | 0 |

Thus a 97.4% reduction was achieved 21 days post-treatment and 100% reduction was achieved 41 days post-treatment.

## EXAMPLE 8 - RAIN AFFECTED TREATMENT

A further advantage of the formulations according to the invention is that they appear to be unaffected by post-treatment rain.

The following example illustrates the efficacy of a formulation according to the invention applied to sheep 4 hours prior to heavy rain.

The following formulation was prepared and administered to romney sheep having 2.5-4.0 cm wool growth.

| | %w/w | mass (kg) |
|---|---|---|
| Diazinon 90S Technical (9051) | 9.878 | 29.634 |
| Teric 12A4 | 1.958 | 5.874 |
| Macro-lex Yellow 3G | 0.743 | 2.229 |
| Corflex 400 | 68.517 | 205.551 |
| Kenmat SC15 | 9.452 | 28.356 |
| TOTAL | 100.000 | 300.700 |
| (diluted 1:5 : application rate 3ml/kg, 45 mg/kg) | | |

Results achieved were as follows:

| Group | % reduction in lice numbers weeks post-treatment | | | | | |
|---|---|---|---|---|---|---|
| | 1 | 2 | 3 | 4 | 5 | 6 |
| Treated sheep | 100 | 100 | 100 | 100 | 100 | 100 |
| Control | 15.5 | 15.4 | 41.5 | 65.3 | 28.0 | 41.5 |

Thus, despite heavy rain post-treatment, formulations according to the invention were effective in eradicating lice infestations. In such weather conditions, sheep are not usually re-treated and this can result in failure to eradiate lice.

It is evident from the above that inclusion of a phthalate extender in pour-on formulations based on organo phosphorus compounds gives substantial advantages over prior art formulations used to treat ectoparasiticidal infestation in sheep.

Furthermore, it is evident that application of such formulations in volumes greater than 2.0 mL/kg body weight of animal also achieves substantially improved effects over prior art formulations designed to ectoparasiticidally treat sheep.

Whilst this invention has been described with respect to <u>Damalinia Ovis</u>; sheep lice, it may also be active against other ectoparasites such as blowfly. Thus the invention should be seen as being not restricted to the particular ectoparasites upon which trials have been conducted.

## Claims

1. Use of an ectoparasiticidal active agent and a non-lanolin dissolving phthalate-comprising solvent-carrier therefor in the preparation of a pour-on ectoparasiticidal formulation for the pour-on treatment of ectoparasitically infested sheep, wherein the ectoparasiticidal active agent is present in said pour-on ectoparasiticidal formulation in an amount of 1-15 % w/w.

2. A use according to claim 1, wherein the active agent is an organophosphorus compound.

3. Use of an ectoparasiticidal active agent and a physiologically acceptable non-lanolin dissolving phthalate-comprising solvent-carrier therefor in the preparation of a pour-on ectoparasiticidal formulation, **characterised in that** said active agent is present in an amount of 1-15 % w/w, for the treatment of ectoparasitically infested sheep by a method comprising pour-on administration of the formulation to a sheep in a volume greater than 1.0 ml 5 per kilo bodyweight of the sheep such that an ectoparasiticidal kill rate as required by the national regulations of the country in which the formulation is to be used is achieved.

4. A use according to claim 3 wherein the volume of ectoparasiticidal formulation administered is greater than 2.0 ml per kilo bodyweight of a sheep to be treated.

5. A use according to any one of claims 3 and 4 wherein the desired ectoparasite kill rate 10 is 100%.

6. A pour-on formulation effective against ectoparasites of sheep comprising an effective amount of at least one active agent in a non-lanolin dissolving solvent-carrier therefor, **characterised in that** the non-lanolin dissolving solvent-carrier: (a) has a viscosity low enough to enable translocation of the at least one active agent around the body of the sheep to be treated, and (b) comprises a phthalate; and further **characterised in that** said active agent is present in an amount of 1 - 15% w/w.

7. A pour-on formulation as claimed in claim 6, further **characterised in that** said active agent and said non-lanolin dissolving-carrier are present in amounts such that the formulation is effective against ectoparasites when delivered in a volume of greater than 1.0 ml per kilo bodyweight of a sheep to be treated.

8. A pour-on formulation as claimed in either of claims 6 and 7, further **characterised in that** said non-lanolin dissolving solvent-carrier is present in an amount of 20 - 85% w/w.

9. A pour-on formulation as claimed in any one of claims 6 to 8, further **characterised in that** said active agent is an organophosphorus compound.

10. A composition comprising an organophosphorus compound in a non-lanolin dissolving solvent-carrier therefor, for use as a pour-on ectoparasiticidal formulation for the treatment of ectoparasitically infested sheep, **characterised in that** the non-lanolin dissolving solvent-carrier: (a) has a viscosity low enough to enable translocation of the at least one active agent around the body of the sheep to be treated, and (b) comprises a phthalate; and further **characterised in that** said active agent is present in an amount of 1 - 15 % w/w.

## Revendications

1. Utilisation d'un agent actif ectoparasiticide et d'un solvant véhicule contenant un phtalate ne dissolvant pas la lanoline dans la préparation d'une formule ectoparasiticide liquide à application externe pour le traitement par application externe de moutons infestés par des ectoparasites, dans laquelle l'agent actif ectoparasiticide est présent dans ladite formule ectoparasiticide à application externe à une teneur de 1 à 15 % p/p.

**2.** Utilisation selon la revendication 1, dans laquelle l'agent actif est un composé organophosphoré.

**3.** Utilisation d'un agent actif ectoparasiticide et d'un solvant véhicule contenant un phtalate ne dissolvant pas la lanoline physiologiquement acceptable dans la préparation d'une formule ectoparasiticide liquide à application externe, **caractérisée en ce que** l'agent actif est présent à une teneur de 1 à 15 % p/p, pour le traitement de moutons infestés par des ectoparasites suivant un procédé comprenant l'administration par application externe de la formulation à un mouton à un volume supérieur à 1,0 ml par kilogramme de poids corporel du mouton de sorte qu'un taux d'élimination ectoparasiticide conforme aux réglementations nationales du pays dans laquelle la formule qui doit être utilisée, soit obtenu.

**4.** Utilisation selon la revendication 3 dans laquelle le volume de formule ectoparasiticide administré est supérieur à 2,0 ml par kilogramme de poids corporel d'un mouton à traiter.

**5.** Utilisation selon l'une quelconque des revendication 3 et 4 dans laquelle le taux d'élimination d'ectoparasite souhaité est 100 %.

**6.** Formule à application externe efficace contre les ectoparasites du mouton comprenant une quantité efficace d'au moins un agent actif dans un solvant véhicule ne dissolvant pas la lanoline, **caractérisé en ce que** le solvant véhicule ne dissolvant pas la lanoline : (a) a une viscosité assez faible pour permettre la translocation de l'au moins un agent actif autour du corps du mouton à traiter, et (b) comprend un phtalate ; et **caractérisée en outre en ce que** ledit agent actif est présent à une teneur de 1 à 15 % p/p.

**7.** Formule à application externe selon la revendication 6, **caractérisée en outre en ce que** ledit agent actif et ledit solvant véhicule ne dissolvant pas la lanoline sont présents à des teneurs telles que la formulation soit efficace contre les ectoparasites quand elle est délivrée à un volume supérieur à 1,0 ml par kilogramme de poids corporel d'un mouton à traiter.

**8.** Formule à application externe selon l'une quelconque des revendications 6 et 7, **caractérisée en outre en ce que** ledit solvant véhicule ne dissolvant pas la lanoline est présent à une teneur de 20 à 85 % p/p.

**9.** Formule à application externe selon l'une quelconque des revendications 6 à 8, **caractérisée en outre en ce que** ledit agent actif est un composé organophosphoré.

**10.** Composition comprenant un composé organophosphoré dans un solvant véhicule ne dissolvant pas la lanoline pour utilisation comme formule liquide à application externe pour le traitement de moutons infestés par des ectoparasites, **caractérisée en ce que** le solvant véhicule ne dissolvant pas la lanoline : (a) a une viscosité assez faible pour permettre la translocation de l'au moins un agent actif autour du corps du mouton à traiter, et (b) comprend un phtalate : et **caractérisée en outre en ce que** ledit agent actif est présent à une teneur de 1 à 15 % p/p.

**Patentansprüche**

**1.** Verwendung eines ektoparasitiziden Wirkstoffes und eines nicht lanolin-lösenden, Phthalat enthaltenden Lösungsmittel-Trägers hierzu zur Herstellung einer ektoparasitiziden Aufgieß-Formulierung für die Aufgießbehandlung von ektoparasitär befallenen Schafen, wobei der ektoparasitizide Wirkstoff in besagter ektoparasitizider Aufgieß-Formulierung in Mengen von 1-15 Gew.-% vorhanden ist.

**2.** Verwendung nach Anspruch 1, wobei der Wirkstoff eine Organophosphorverbindung ist.

**3.** Verwendung eines ektoparasitiziden Wirkstoffes und eines physiologisch verträglichen, nicht lanolin-lösenden, Phthalat enthaltenden Lösungsmittel-Trägers hierzu zur Herstellung einer ektoparasitiziden Aufgieß-Formulierung, **dadurch gekennzeichnet, daß** besagter Wirkstoff in Mengen von 1-15 Gew.-% vorhanden ist, zur Behandlung ektoparasitär befallener Schafe mittels eines Verfahrens, das die Aufgießverabreichung der Formulierung an Schafe umfaßt, in einem Volumen, das größer als 1,0 ml pro kg Körpergewicht des Schafes ist, so daß eine ektoparasitizide Abtötungsrate erreicht wird, wie sie von den nationalen Vorschriften des Landes gefordert wird, in dem die Formulierung verwendet werden soll.

**4.** Verwendung nach Anspruch 3, wobei das Volumen der verabreichten ektoparasitiziden Formulierung größer ist als 2,0 ml pro kg Körpergewicht des zu behandelnden Schafes.

**5.** Verwendung nach einem beliebigen der Ansprüche 3 und 4, wobei die gewünschte Ektoparasiten-Abtötungsrate 100 % ist.

**6.** Aufgieß-Formulierung, wirksam gegen Ektoparasiten von Schafen, umfassend eine wirksame Menge von mindestens einem Wirkstoff in einem nicht lanolin-lösenden Lösungsmittel-Trägex hierzu, **dadurch gekennzeichnet, daß** der nicht lanolin-lösende Lösungsmittel-Träger (a) eine Viskosität besitzt, die niedrig genug ist, um eine Verteilung von mindestens einem Wirkstoff um den Körper des zu behandelnden Schafes ermöglicht, und (b) ein Phthalat enthält; und darüber hinaus **dadurch gekennzeichnet ist, daß** der besagte Wirkstoff in einer Menge von 1-15 Gew.-% vorhanden ist.

**7.** Aufgieß-Formulierung wie in Anspruch 6 beansprucht, ferner **dadurch gekennzeichnet, daß** der besagte Wirkstoff und der besagte nicht lanolin-lösende Träger in Mengen vorhanden sind, so daß die Formulierung gegen Ektoparasiten wirksam ist, wenn sie in einem Volumen von mehr als 1,0 ml pro kg Körpergewicht des zu behandelnden Schafes verabreicht wird.

**8.** Aufgieß-Formulierung wie in einem der Ansprüche 6 und 7 beansprucht, ferner **dadurch gekennzeichnet, daß** der besagte nicht lanolin-lösende Lösungsmittel-Träger in Mengen von 20-85 Gew.-% vorhanden ist.

**9.** Aufgieß-Formulierungen wie in einem beliebigen der Ansprüche 6 bis 8 beansprucht, ferner **dadurch gekennzeichnet, daß** der besagte Wirkstoff eine Organophosphorverbindung ist.

**10.** Zusammensetzung, umfassend eine Organophosphorverbindung in einem nicht lanolin-lösenden Lösungsmittel-Träger hierzu, zur Verwendung als ektoparasitizide Aufgieß-Formulierung für die Behandlung von ektoparasitär befallenen Schafen, **dadurch gekennzeichnet, daß** der nicht lanolin-lösende Lösungsmittel-Träger (a) eine Viskosität besitzt, die niedrig genug ist, um eine Verteilung von mindestens einem Wirkstoff um den Körper des zu behandelnden Schafes zu ermöglichen, und (b) ein Phthalat enthält; und ferner **dadurch gekennzeichnet ist, daß** das der besagte Wirkstoff in Mengen von 1-15 Gew.-% vorhanden ist.